# EUROPEAN PATENT SPECIFICATION

(11) **EP 3 656 369 B1**
(45) Date of publication and mention of the grant of the patent: **30.08.2023**
(21) Application number: 19210429.7
(22) Date of filing: 20.11.2019
(51) Int. Cl.: A61K 8/02, A61K 8/26, A61K 8/34, A61K 8/81, A61K 8/891, A61K 8/36, A61K 8/73, A61Q 19/00

(54) **COMPOSITION FOR IMPREGNATING PERSONAL CARE WIPES COMPRISING A LAYERED SILICATE**
ZUSAMMENSETZUNG ZUR IMPRÄGNIERUNG VON TÜCHERN ZUR KÖRPERPFLEGE ENTHALTEND EIN SCHICHTSILIKAT
COMPOSITION POUR L'IMPRÉGNATION DE LINGETTES DE SOINS PERSONNELS COMPRENANT UN SILICATE STRATIFIÉ

(30) Priority: 22.11.2018 NL 2022049
(43) Date of publication of application: 27.05.2020
(73) Proprietor: Arion Holding B.V., 6422 RE Heerlen (NL)
(72) Inventor: Joosten, Erik, 3000 Helsingør (DK); De Kogel, Christina Emmerentia, 3545 Loksbergen (BE)
(74) Representative: Brouwer, Hendrik Rogier

(56) References cited:
- WO-A1-2008/097223
- WO-A1-2014/208093
- JP-A- 2008 050 271
- US-A1- 2014 004 166
- US-A1- 2018 168 316
- DATABASE GNPD [Online] MINTEL; 22 August 2017 (2017-08-22), anonymous: "Body Sheet UV Sun Screen SPF 25 PA++", XP055589385, retrieved from www.gnpd.com Database accession no. 5044725

## Description

### FIELD OF THE INVENTION

The invention relates to a composition that is preferably used in an article for personal care. The invention further relates to an article for personal care comprising the composition according to the present invention, and to a process for preparing the article for personal care.. The invention is not limited to a specific article. Suitable articles may comprise a sheet or wipe, but other geometries may also be possible, such as a glove to cover a hand, or a cap to cover a user's hair.

### BACKGROUND

A wipe for personal care is generally known for cleaning and/or protecting a skin. The wipe comprises a nonwoven or textile carrier and a composition, such as a liquid or a thin lotion, for cleaning and/or protecting the skin. A disadvantage of the known composition is that it is a liquid having low viscosity, which, when wiping the cleaning wipe including the composition over the skin, has a relatively low substantivity to the skin only, and possibly also a low visibility on the skin.

Other than aqueous solutions, wet wipes may be impregnated with more substantial lotions, like light cream emulsions. Such creams are designed to impart more protective or caring characteristics, which may be attributed in part to their oil phase, and is typically associated with the use of fatty acid esters.

A sunscreen composition is known from WO 2008/097223 A1. It is used for impregnating a wipe substrate or as a sunscreen spray. The disclosed composition comprises an oil phase containing a UV-absorbing organic sunscreen active. The oil phase is dispersed stably as emulsion droplets in a water phase that contains i) a booster for the sun protection factor (SPF), comprising a combination of water-dispersible particulate materials, one of which is smectite clay, and a water-soluble or water-dispersible phenolic polymer; and ii) a water-soluble or water-dispersible polymer having a weak acid group, a weight average molecular weight of 1, 000 - 100, 000 Dalton, and an anionic charge density of no less than 4 milliequivalent per gram of the polymer.

US 2014/004166 A1 discloses an emulsion suitable for incorporation into wet wipes used to clean urine and faecal matter from the skin. The emulsion includes silicone oil and clay. Phase separation is prevented with an emulsification system that includes a gum blend and either a Gemini surfactant restrained to the oil phase or a glucoside-based emulsifier. The gum blend includes at least one gum and propylene glycol alginate.

Viscosity (η) is a measure of a fluid's internal flow resistance. It may be defined as the resistance, which a fluid shows when being deformed, and it gives information on how thick a fluid is and how easily it flows.

The inclusion of functional additives and excipients, including active ingredients, emollients, and humectants and so on, usually results in a thicker lotion with higher viscosity. In prior art, such lotions would commonly be based on emulsion technology, for example oil-in-water or water-in-oil emulsion formulas. Typical emulsifiers would include esters of fatty acids such as stearic acid, lauric acid, palmitic acid, and myristic acid. These fatty acids could for example be esterified with glycerin or polyethylene glycols.

As cream emulsions usually have a considerably higher viscosity than aqueous solutions, traditional impregnation processes at room temperature may be less appropriate. Traditional emulsions require hot processing, and may then be used for impregnation while hot or during the cooling phase, to take advantage of reduced viscosity while hot to overcome impregnation difficulties, thus obtaining even impregnation of the nonwoven material of the wipe.

High viscosity in lotions results in critical technical challenges during pumping of the lotion through the piping system and the impregnation process. Viscous lotions are not easily pumped through the piping system, or cannot be pumped at all. Viscous lotions more easily clog the spray nozzles, and viscous lotions tend to have less favourable spreading characteristics, thus potentially resulting in uneven dispersion of the lotion in the nonwoven cloth. Elevated viscosity of lotions for impregnation of nonwovens is therefore not preferred.

Hot process manufacturing has higher manufacturing cost implications, and has a larger energy footprint than cold process manufacturing. Cold processing represents savings in time and energy, and dedicated equipment with heating and cooling facilities are not required. Cold process manufacturing of stable traditional cream emulsions, such as the stearates types, is unlikely.

Impregnation of nonwoven cloth materials usually involves extensive piping systems, with in-line impregnation nozzles at the end. The flow rate of the impregnation lotion through the nozzles has to be sufficiently high, to keep up with the velocity of the nonwoven substrate material. Both impregnation lotion and cloth material are selected such that the lotion is readily absorbed, evenly dispersed, and adequately retained by the nonwoven material.

Embodiments of the invention aim to provide a composition usable for personal care such as for cleaning and/or protecting a skin, wherein an article including the composition is easily prepared and wherein the composition attributes and/or enhances a cleaning and /or protection of the skin. Particular embodiments aim to provide a process for preparing the article, wherein the article including the composition is easily prepared at a lower temperature such as room temperature and wherein the composition attributes and/or enhances a cleaning and/or protection of the skin. Other particular embodiments aim to provide a composition, wherein the composition further attributes and/or enhances a moisturizing behavior of the skin, which has been cleaned by said composition.

### SUMMARY OF THE INVENTION

According to a first aspect of the invention there is provided a composition in accordance with claim 1. The composition comprises a layered silicate, an acrylate polymer, a preservative compound, a humectant compound, further a hyaluronic acid or a salt derivative thereof, and water. According to the invention, the weight ratio between the layered silicate and the acrylate polymer in the composition is in the range from 2.6 : 1.0 to 4.3 : 1.0, and the composition has a thixotropy according to ISO 3219 at a temperature of 23°C of 100 to 1000 Pals.

The current invention has addressed the challenges, utilizing a novel balanced mixture of thickening ingredients: layered silicate and acrylate polymer. The composition is based on hydrogel technology, which suspends caring ingredients rather than emulsifying them. The thickening system of the composition has been designed to be shear thinning, thus allowing for adequate manufacturing pumping conditions and nonwoven impregnation upon nozzle-spraying. The suspension of the layered silicate in combination with the acrylate polymer forms a hydrogel base, which provides a thickening effect and a shear thinning behavior to the composition. In particular, the layered silicate in the acrylate polymer forms a suspension system inside the composition. The composition is thickened in a rest state, when no shear is applied. During manufacturing of an article comprising the composition, the composition may easily be transported, e.g. by pumping, and applied into a textile carrier of the article due to its shear thinning behavior. In this way, the article including the composition is easily prepared.

During use of the article by wiping it over the skin, the composition forms a relatively abundant or rich layer on the skin, due to its relatively high viscosity during a wiping movement. In this way, the composition enhances a cleaning and/or protection of the skin.

In embodiments, the layered silicate may be a clay mineral and may be a synthetic layered silicate. Clay minerals are hydrous aluminum phyllosilicates. Preferably, the layered silicate is a phyllosilicate.

Layered silicate can be classified as 1:1 or 2:1, this originates because they are fundamentally built of tetrahedral silicate sheets and octahedral hydroxide sheets. A 1:1 layered silicate would consist of one tetrahedral sheet and one octahedral sheet, and examples would be kaolinite and serpentine. A 2:1 layered silicate consists of an octahedral sheet sandwiched between two tetrahedral sheets, and examples are talc, vermiculite and montmorillonite.

In an exemplary embodiment, the layered silicate is a smectite. The smectite group includes dioctahedral smectites such as montmorillonite, nontronite and beidellite and trioctahedral smectites, for example saponite.

In a particular embodiment, the smectite is selected from the group consisting of bentonite, hectorite, montmorillonite, nontronite, beidellite, saponite, stevensite and mixtures thereof. In a preferred embodiment, the smectite is a bentonite. Bentonite is an absorbent aluminum phyllosilicate layered silicate. In an example, the smectite is a magnesium aluminum silicate.

In another aspect of the present invention, a hydrogel suspension composition is provided containing a magnesium aluminum silicate and an acrylamidepolymer, such as polyacrylate crosspolymer-11.

In an exemplary embodiment, the amount of the layered silicate is at least 0.3 wt.%, preferably at least 0.5 wt.%, more preferably at least 0.8 wt.%, based on the total weight of the composition. When referring to the total weight of the composition throughout the description, the weight of the water is included. Said amount enhances a control on the shear thinning behavior of the composition.

In an exemplary embodiment, the amount of the layered silicate is at most 5.0 wt.%, preferably at most 2.5 wt.%, more preferably at most 1.8 w-t.%, based on the total weight of the composition.

In an exemplary embodiment, the acrylate polymer comprises acrylamide units. The acrylamide units of the acrylate polymer enhance the control on the shear thinning behavior of the composition in combination with the selected layered silicate. In a particular embodiment, a part of the acrylamide units comprises a sulphonic acid group, which is modified by an ammonium salt. The sulphonic acid groups of the acrylate polymer, which are modified by an ammonium salt, further enhance the control on the shear thinning behavior of the composition in combination with the selected layered silicate. Applying a poly(vinyl-acrylate) copolymer to the composition is less preferred. More preferably, an embodiment of the composition does not comprise an acryloyldimethyltaurate copolymer, such as Aristoflex AVC (INCI: Ammonium Acryloyldimethyltaurate/VP Copolymer).

In an exemplary embodiment, the amount of the acrylate polymer is at most 1.0 wt.%, preferably at most 0.5 wt.%, based on the total weight of the composition. Said amount enhances a control on the shear thinning behavior of the composition.

In an exemplary embodiment, the amount of the acrylate polymer is at least 0.01 wt.%, preferably at least 0.1 wt.%, based on the total weight of the composition.

According to the invention, the ratio between the layered silicate and the acrylate polymer based on their weight is in the range from 2.6: 1.0 to 4.3 : 1.0. The weight ratio between the layered silicate and the acrylate polymer enhances a formation of a relatively abundant layer of the composition on the skin.

According to the invention, the composition further comprises hyaluronic acid or a salt derivative thereof. The addition of hyaluronic acid or its salt has been shown to further strengthen the viscosity of the composition, without negatively affecting either yield point or thixotropy of the composition. The hyaluronic acid may be a natural glucosaminoglycan having a high molecular weight. The hyaluronic acid or a salt derivative thereof may also act as an effective moisturizer for the skin, which easily forms a film on the skin due to its high molecular weight. In this way, a moisturizing effect of the composition is further enhanced.

In an exemplary embodiment, the amount of said hyaluronic acid including the salt derivatives thereof is up to 0.5 wt.% based on the total weight of the composition. The hyaluronic acid and/or its salt derivatives preferably have a molecular weight of at least 800 kDa, more preferably of at least 1500 kDa.

In an exemplary embodiment, the salt derivative of the hyaluronic acid is selected from at least one of a sodium salt, a potassium salt, and a zinc salt. The salt derivative of the hyaluronic acid may suitably be selected to improve the moisturizing effect of the hyaluronic acid in the composition. In a particular embodiment, the salt derivative of the hyaluronic acid is a zinc salt thereof. The zinc salt derivative of the hyaluronic acid additionally supports skin recovering or skin healing.

In an exemplary embodiment, the composition has a dynamic viscosity exceeding 50 mPas, preferably exceeding 100 mPas. The viscosity, the yield point and the thixotropy of a composition are measured according to ISO 3219. The measurement is carried out at 23.0°C. Compositions with a dynamic viscosity up to typically 50 or 100 mPas can be pumped through the piping system and nozzles without any specific problems. In case of a dynamic viscosity exceeding typically 100 mPas, thixotropy may be needed to enable processing the lotion in the piping and nozzle system.

In an exemplary embodiment, the composition has a yield point between 25 and 250 Pa, preferably between 50 and 150 Pa. The viscosity, the yield point and the thixotropy of a composition are measured according to ISO 3219. The measurement is carried out at 23.0°C. Preferably, the measured yield point exceeds 50 Pa.

According to the invention, the composition has a thixotropy of 100 to 1000 Pals, preferably between 250 and 750 Pals. The viscosity, the yield point and the thixotropy of a composition are measured according to ISO 3219. The measurement is carried out at 23.0°C. Preferably, the thixotropy is around 500 Pa/s.

In an exemplary embodiment, the humectant compound comprises at least one of the group consisting of an alkyl glycol, such as propylene glycol, dipropylene glycol, butylene glycol, pentylene glycol, etc., glycerin, and glycerin derivatives. The humectant compound attributes to or enhances a moisturizing effect of the skin.

In an exemplary embodiment, the composition additionally comprises a poly(dimethyl)siloxane polymer. The poly(dimethyl)siloxane polymer has a low surface tension. The poly(dimethyl)siloxane polymer is substantially immiscible to water. The poly(dimethyl)siloxane polymer preferably has a viscosity around 100 - 1000 mPas, more preferably about 350 mPas. In an embodiment, the poly(dimethyl)siloxane polymer is dimethicone. The poly(dimethyl)siloxane polymer enhances a soft feel of the wipe as it enhances a lubricant effect, and attributes to protecting a skin after cleaning by forming a stable film on said skin.

In exemplary embodiments, the composition may additionally comprise an emulsifying compound. The emulsifying compound supports to form an emulsion in the composition containing water. In particular, the emulsifying compound may form an emulsion for containing the poly(dimethyl)siloxane polymer. The emulsifying compound comprises at least one component of the group consisting of fatty acid derivate of triglyceride, such as caprylic triglyceride and capric triglyceride, a glucoside, such as lauryl glucoside, a lactylate, such as sodium lauroyl lactylate and sodium stearoyl lactylate, a monoglyceride, such as glyceryl stearate, glyceryl citrate, glyceryl lactate, glyceryl linoleate, glyceryl oleate, and glyceryl caprylate, a fatty alcohol, such as cetearyl alcohol, a fatty acid derivate of a polyglycerol, such as polyglyceryl-4-cocoate and polyglyceryl-3-caprate, and mixtures thereof. The emulsifying compound may suitably be selected such that the components thereof are biodegradable.

In an exemplary embodiment, the composition additionally comprises a preservative compound. The preservative compound prevents decomposition of the composition by microbial growth or by undesirable chemical changes. The preservative compound may be selected from the approved preservative compounds listed in Annex V of Regulation (EC) No 1223/2009 of the European Parliament, entitled the Cosmetic Product Regulation (CPR), online available at: https://eur-lex.europa.eu/legal-content/EN/ALL/?uri=CELEX%3A32009R1223.

In more preferred embodiments, the preservative compound is selected from at least one of a sorbic acid, a derivative of sorbic acid, benzoic acid, a derivative of benzoic acid, lactic acid, propionic acid, one or more parabens, and phenoxyethanol. The preservative compound prevents degradation of the composition by bacteria, moulds and fungi.

In another aspect of the present invention a process is provided for preparing an article comprising the steps:
a. providing i. a textile carrier; ii. a composition according to the present invention; and
b. impregnating the textile carrier by the composition at a temperature ranging from 5 °C to 50 °C.

The advantages of the present invention become more apparent in case the impregnating step of the textile carrier by the composition may be carried out at a relatively low temperature. The composition may easily be applied into the textile carrier by impregnation step due to the shear thinning behavior of the composition. In particular, the impregnating step is carried out at a temperature of 5 °C to 50 °C, preferably at a temperature of 10 °C to 40 °C, more preferably at a temperature of 15 °C to 30 °C.

Due to the desirable rheological properties of the composition, applying pressure to the composition during impregnation of the textile carrier with the composition may not be needed. A preferred embodiment of the invention therefore provides a process wherein step b. is carried out substantially pressureless, preferably by spraying the composition onto the textile carrier. This embodiment provides advantages in terms of process efficiency.

The textile carrier (component i.) may be a woven fabric, or may be a non-woven fabric. The textile carrier may comprise a fabric of natural fibers and/or synthetic fibers. A woven fabric may be made by weaving together fibers of silk, cotton, polyester, wool, and similar materials to form an interlocking matrix of loops. Non-woven fabrics, on the other hand, are made by a process that presses a single sheet of material from a mass of separate fibers. Fibers, such as cotton, cellulose and regenerated cellulose, may be used in this process, as well as plastic resins like polyester, polyethylene, and polypropylene, or mixtures thereof. The textile carrier may be presented as a sheet or wipe, but other geometries may be applicable, such as a glove to cover a hand, or a cap to cover a user's hair. Corresponding articles then have about the same geometry. The loading may be adapted accordingly, wherein the loading represents the weight ratio of the textile carrier relative to the composition.

The composition (component ii.) may be any composition according to the present invention. The composition (component ii.) may be suitably selected for impregnating the textile carrier (component i.).

In an exemplary embodiment, in step a. the composition ii. is provided inside a container, the process additionally comprising the step: c. pumping the composition from the container towards the textile carrier at a temperature of 5 °C to 50 °C. During the pumping process the composition is easily transported, such as through supply tubes, towards the textile carrier due to its shear thinning behavior. In particular, the pumping step is carried out at a temperature of 5 °C to 50 °C, preferably at a temperature of 10 °C to 40 °C, more preferably at a temperature of 15 °C to 30 °C.

In another aspect of the present invention an article, preferably a wipe, is provided, comprising a textile carrier and the composition according to the present invention.

In another aspect of the present invention an article for use according to the amended set of claims is provided. The article including the composition according to the present invention supports to apply a relatively abundant layer of the composition onto a skin when wiping the article over the skin. The relatively abundant layer of the composition attributes and/or enhances a moisturizing effect of the skin and supports a protection of the skin.

### EXAMPLES

The following examples further illustrate the invention. In the following, all amounts given in the Tables are parts by weight if not indicated otherwise.

### Raw materials

| Aq | Water |
|---|---|
| SMEC 1 | Veegum Ultra, Bentonite clay (manufacturer Vanderbilt Minerals LLC) |
| ACRPOL 1 | Aristoflex Velvet, acrylamidepolymer (manufacturer Clariant International Ltd) |
| PR-GL | Propylene glycol |
| GLY | Glycerin |
| HYAL 1 | Hyaluronic acid, sodium salt of |
| DIMET | Dimethicone |
| EMUL 1 | Emulsogen CCT (manufacturer Clariant International Ltd) |
| PRES | Suitable preservative mix, including e.g. sorbic acid, potassium sorbate |

The SMEC 1, Veegum Ultra, consists of magnesium aluminum silicate, preferably variants with fast solubility in water. The DIMET dimethicone has a viscosity of 100-1000 cP, and preferably 350 cP. The EMUL 1, Emulsogen CCT, contains a mixture of Caprylic / Capric Triglyceride (and) Aqua (and) Glycerin (and) Laureth-23 (and) Behenyl Alcohol (and) Disodium Ethylene Dicocamide (and) Glyceryl Stearate (and) Sodium Lauroyl Lactylate (and) Glyceryl Stearate Citrate.

### Nonwoven materials

Wet wipes and associated nonwoven products are designed to release lotions when used, and to absorb and retain during the manufacturing process and upon storage. The used lotions are mostly water based. The nonwoven materials used must therefore contribute to an adequate balance of hydrophobic and hydrophilic properties, thus ensuring good impregnation, product stability, and product functionality when used.

The nonwoven material consists of natural or man-made polymers, or a mixture thereof. Typical natural materials would include cotton, wood pulp, cellulose, silk and wool, typical man-made materials include nylon, polyester, polyethylene, polypropylene, polylactic acid, regenerated cellulose and acrylics. The nonwoven material is preferably a blend of polyester first fibers and regenerated cellulose second fibers. Preferably, the regenerated cellulose is lyocell. The fibers are blended by mechanical entanglement, such as spun lacing or needle punching. Preferably, the hydrophobic polyester fibers constitute at least 40% of the blend by weight, while the hydrophilic fibers constitute at most 60% of the blend by weight. The nonwoven material may or may not be cross-lapped.

### Measurement methods

### Viscosity. Yield point and thixotropy

When the dynamic viscosity of a composition is too high to enable pumping it through a piping system, an alternative would be to select or create a composition that becomes sufficiently fluid when force is applied.

The yield point (τ0, also called yield stress) is the lowest shear-stress value above which a material will behave like a fluid, and below which the material will act like a solid. The yield point is the minimum force that must be applied to a material so that it starts to flow. If the yield stress is too high, the composition will not flow under desired manufacturing conditions, and will remain solid. If the yield stress is too low, flow will occur too easily. This would be interesting for pumping and impregnating, but the impregnated nonwoven product will be unstable.

Thixotropy (Δ) may be defined as the continuous decrease of apparent viscosity with time under shear and the subsequent recovery of the viscosity when the flow is discontinued.

Thixotropic nature of a fluid is characterized by a hysteresis curve. The surface area of the hysteresis curve is linear proportional to the magnitude of thixotropy. A small surface area means low thixotropy, and denotes rapid return of the composition to its solid state. The thixotropy may be too low to properly impregnate the nonwoven material. A large surface area means high thixotropy, and denotes slow return of the composition to its solid state. The composition remains too runny for too long, resulting in suboptimal dispersion of the composition in the nonwoven cloth.

Measuring thixotropy (Δ), yield stress (τ0) and viscosity (η) requires a rheometer. A typical rheometer, used for characterizing this invention, is the Haake Viscotester IQ of Thermo Fisher Scientific Inc., which is a rotational rheometer equipped with a coaxial cylinder geometry (for instance the CC25 DIN/Ti geometry). The geometry operates according to ISO standard 3219.

The viscosity, the yield point and the thixotropy of a composition are measured according to ISO 3219. The measurement is carried out at 23.0°C. The viscosity is determined at a shear rate of 1/100 s⁻¹ over 60 s.

The yield point is determined after the composition has had sufficient time to recover any thixotropic structure. The yield point is expressed in [Pa] and determined at a constant shear rate of at least 1/10000 s⁻¹ as a logarithmic shear stress curve of 0.001-500 Pa over 60 s.

The thixotropy is determined quantitatively after the composition has had sufficient time to recover any thixotropic structure by determining the shear stress vs. shear rate hysteresis loop, at a shear rate of 1/10000 - 1000 s⁻¹ over 60 s (linear, forward curve), 1000 s⁻¹ over 30 s, and 1000 - 1/10000 s⁻¹ over 60 s (linear, backward curve). The thixotropy is expressed in [Pals] and calculated as the difference in area under the curve between the forward curve and the backward curve.

### Application and Performance

### Preparation of the composition

The preparation of the composition is separated into several steps for better clarity. The steps are:
1. Provide at least 75 weight-% of the water
2. Add the layered silicate component and the acrylpolymer in the water
3. Mix the mixture of step 2. to form a stable suspension of the layered silicate component and the acrylpolymer in water
04. Optionally add preservatives to the mixture of step 3.
5. Optionally prepare a premixture of the dimethicone and the emulsifier compound in water, and add the premixture of the dimethicone and the emulsifier compound in water to the mixture of step 4 or step 3.
6. Add the humectant, optionally including other ingredients, such as a hyaluronic acid component, to 5 the mixture of step 3., 4. or 5.
7. Add water to the mixture such that the total amount of water is supplied.

The following compositions were prepared according to the steps explained above:

**Table 1: compositions**

| Ingredient | Example 1 [wt %] | Example 2 [wt %] | Example 3 [wt %] | Example 4 [wt %] | Example 5 [wt %] | Example 6 [wt %] | Example 7 [wt %] |
|---|---|---|---|---|---|---|---|
| SMEC 1 | 1.30 | 1.30 | 1.00 | - | 1.30 | 1.30 | 1.30 |
| ACRPOL 1 | 0.40 | 0.40 | 0.40 | 0.40 | 0.30 | 0.50 | - |
| PR-GL | 2.00 | 2.00 | 2.00 | 2.00 | 2.00 | 2.00 | 2.00 |
| GLY | 1.00 | 1.00 | 1.00 | 1.00 | 1.00 | 1.00 | 0.50 |
| HYAL 1 | - | 0.05 | 0.05 | 0.05 | 0.05 | 0.05 | 0.05 |
| DIMET | 3.00 | 3.00 | 3.00 | 3.00 | 3.00 | 3.00 | 3.00 |
| EMUL 1 | 3.00 | 3.00 | 3.00 | 3.00 | 3.00 | 3.00 | 3.00 |
| PRES | 0.70 | 0.70 | 0.70 | 0.70 | 0.70 | 0.70 | 0.70 |
| Aq* | Qs | Qs | Qs | Qs | Qs | Qs | Qs |
| Total | 100.00 | 100.00 | 100.00 | 100.00 | 100.00 | 100.00 | 100.00 |

Only example 2 falls within the scope of the claimed invention.

Qs: the amount of water (Aq) is such that the total of the ingredients is 100.00 wt%.

The total weight of the suspension system (the layered silicate and the acrylpolymer) and the weight ratio between the layered silicate and the acrylpolymer for the Examples 1 - 7 is shown in the following Table:

**Table 2: suspension system [wt%] and weight ratio [/]**

| Ingredient | Example 1 | Example 2 | Example 3 | Example 4 | Example 5 | Example 6 | Example 7 |
|---|---|---|---|---|---|---|---|
| suspension system [wt. %] | 1.70 | 1.70 | 1.40 | 0.40 | 1.60 | 1.80 | 1.30 |
| Weight Ratio [/] | 3.25 | 3.25 | 2.50 | 0.00 | 4.33 | 2.60 | » |

### Preparation of the wipe

The preparation of the wipe is separated into several steps for better clarity. The steps are:
1. Provide a textile carrier
2. Provide a composition according any one of the examples in a container
3. Pump the composition towards the textile carrier at a temperature of about 10 - 30 °C
4. Apply the composition onto the textile carrier, while shearing the composition
5. Let the composition impregnate the textile carrier at a temperature of about 10 - 30 °C

The textile carrier used in these tests is a non-woven fabric, such as a fabric containing cotton fibers.

Each of the compositions according to the examples 1 - 6 is used to impregnate a non-woven fabric. Each of the compositions shows a shear thinning behavior, which makes them suitable to pump the composition towards the textile carrier at a temperature of about 15 - 30 °C and to impregnate the textile carrier at a temperature of about 15 - 30 °C.

Additionally, each of the compositions is sufficiently thick at a temperature of about 15 - 30 °C to provide a sufficient abundant layer on a skin, when wiping the wipe including the composition over the skin.

Each of the wipes having a composition according to the examples 1 - 6 is evaluated on skin feel sensation, when wiping the wipe over the skin. A skin feel is a sense of softness of the wipe when manually wiping the wipe over the skin. The wiping test was performed at ambient temperature of about 15 - 30 °C.

Examples 1, 2 and 6 showed an appropriate skin feel. Examples 3-5 showed a reasonable, but lesser, skin feel. It is assumed that the weight ratio between the layered silicate and the acrylpolymer is relevant for the skin feel of the wipe. In these examples, a higher weight ratio of higher than 2.5 correlates to a more thickened composition at a temperature of about 15 - 30 °C.

The viscosity, yield point and thixotropy index are shown in Table 3.

**Table 3: viscosity, yield point and thixotropy index**

| | Example 1 | Example 2 | Example 3 | Example 4 | Example 5 | Example 6 | Example 7 |
|---|---|---|---|---|---|---|---|
| Viscosity (η) (mPas) | 124 | 178 | 159 | 155 | 113 | 226 | unstable |
| Yield Point (τ0) (Pa) | 74 | 115 | 110 | 114 | 48 | 22 | unstable |
| Thixotropic (Δ) (Pa/s) | 500 | 383 | 2418 | 1943 | 0 | 2310 | unstable |

The results show that the layered silicate, preferably the smectite, plays a role in reducing the thixotropy. A composition containing less (Example 3) or no (Example 4) layered silicate has a thixotropy which is too high for this type of application. The composition stays "runny" for too long, resulting in the composition not sticking to but going through the textile upon impregnation.

Additionally, when the composition has less (Example 5) or no (Example 7) acrylate polymer, the composition either does not have a thixotropy behaviour (see Example 5) or does not have a stable viscosity, yield point and thixotropy (Example 7). Both are unsuitable for application in impregnated textiles at room temperature. Example 6 shows that too much acrylate polymer disturbs the balance with the layered silicate, resulting in increased dynamic viscosity combined with a thixotropy which is too high for this type of impregnation application. Addition of Sodium Hyaluronate (Example 2) further optimises dynamic viscosity, without negatively affecting the thixotropy. Additionally, the thixotropic index increases when the weight ratio is close to 2.60 and lower or the weight ratio is at least 4.30. The thixotropic index is controlled to be in the range 100 to 1000 Pa/s, preferably between 250 and 750 Pa/s.

## Claims

1. A composition comprising at least a layered silicate, an acrylate polymer, a preservative compound, a humectant compound and water, wherein the composition further comprises hyaluronic acid or a salt derivative thereof, wherein the weight ratio between the layered silicate and the acrylate polymer is in the range from 2.6 : 1.0 to 4.3 : 1.0, and the composition has a thixotropy according to ISO 3219 at a temperature of 23°C of 100 to 1000 Pals.

2. The composition according to claim 1, wherein the amount of the hyaluronic acid or the salt derivative thereof is up to 0.5% wt.% based on the total weight of the composition.

3. The composition according to claim 1 or 2, wherein the molecular weight of the hyaluronic acid or the salt derivative thereof is at least 800 kDa, and more preferably at least 1500 kDa.

4. The composition according to any one of the preceding claims, wherein the salt derivative of the hyaluronic acid is selected from at least one of a sodium salt, a potassium salt, and a zinc salt.

5. The composition according to any one of the preceding claims, wherein the layered silicate is a smectite, preferably selected from the group consisting of bentonite, hectorite, montmorillonite, nontronite, beidellite, saponite, stevensite and mixtures thereof, more preferably a bentonite.

6. The composition according to any one of the preceding claims, wherein the amount of the layered silicate is at least 0.3 wt-%, preferably at least 0.5 wt-%, more preferably at least 0.8 wt-%, based on the total weight of the composition.

7. The composition according to any one of the preceding claims, wherein the acrylate polymer comprises acrylamide units, preferably a part of the acrylamide units comprising a sulphonic acid group, which is modified by an ammonium salt.

8. The composition according to any one of the preceding claims, wherein the amount of the acrylate polymer is at most 1.0 wt-%, preferably at most 0.5 wt-%, based on the total weight of the composition.

9. The composition according to any one of the preceding claims, having a dynamic viscosity according to ISO 3219 at a temperature of 23°C exceeding 50 mPas, preferably exceeding 100 mPas.

10. The composition according to any one of the preceding claims, having a yield point according to ISO 3219 at a temperature of 23°C between 25 and 250 Pa, preferably between 50 and 150 Pa.

11. The composition according to any one of the preceding claims, having a thixotropy according to ISO 3219 at a temperature of 23°C of between 250 and 750 Pa/s.

12. The composition according to any one of the preceding claims, further comprising a poly(dimethyl)siloxane polymer.

13. The composition according any one of the preceding claims, further comprising an emulsifying compound, wherein the emulsifying compound comprises at least one component of the group consisting of fatty acid derivate of triglyceride, a glucoside, a lactylate, a monoglyceride, a fatty alcohol, a fatty acid derivate of a polyglycerol, and mixtures thereof.

14. An article for personal care, comprising a textile carrier and a composition according to any one of the preceding claims.

15. The article according to claim 14 for use in at least one of skin moisturisation or hydration, skin regeneration, restoration of skin barrier, relief or prevention of skin blemishes, treating age related skin conditions, relief or prevention of skin infections, skin wound healing, relief or prevention of dermatitis, or for enhancing anti-acne properties of the skin.

16. Process for preparing an article for personal care, comprising the steps of:
a. providing
i.a textile carrier;
ii.a composition according to any one of the claims 1 - 13; and
b. impregnating the textile carrier with the composition at a temperature ranging from 5°C to 50°C.

17. Process according to claim 16, wherein step b. is carried out substantially pressureless, preferably by spraying the composition onto the textile carrier.

## Patentansprüche

1. Eine Zusammensetzung, aufweisend mindestens ein Schichtsilikat, ein Acrylatpolymer, eine konservierende Verbindung, eine feuchtigkeitsspendende Verbindung sowie Wasser, wobei die Zusammensetzung ferner Hyaluronsäure oder ein Salzderivat davon aufweist, wobei das Gewichtsverhältnis zwischen dem Schichtsilikat und dem Acrylatpolymer im Bereich von 2,6:1,0 bis 4,3:1,0 liegt und die Zusammensetzung eine Thixotropie gemäß ISO 3219 bei einer Temperatur von 23°C von 100 bis 1000 Pa/s aufweist.

2. Die Zusammensetzung nach Anspruch 1, wobei die Menge der Hyaluronsäure oder deren Salzderivat bis zu 0,5 Gew.-%, bezogen auf das Gesamtgewicht der Zusammensetzung, beträgt.

3. Die Zusammensetzung nach Anspruch 1 oder 2, wobei das Molekulargewicht der Hyaluronsäure oder deren Salzderivat mindestens 800 kDa und vorzugsweise mindestens 1500 kDa beträgt.

4. Die Zusammensetzung nach einem der vorherigen Ansprüche, wobei das Salzderivat der Hyaluronsäure ist ausgewählt aus mindestens einem Natriumsalz einem Kaliumsalz und einem Zinksalz.

5. Die Zusammensetzung nach einem der vorherigen Ansprüche, wobei das Schichtsilikat ein Smektit darstellt, vorzugsweise ausgewählt aus der Gruppe bestehend aus Bentonit, Hectorit, Montmorillonit, Nontronit, Beidellit, Saponit, Stevensit und Mischungen davon, besonders bevorzugt ein Bentonit.

6. Die Zusammensetzung nach einem der vorherigen Ansprüche, wobei die Menge des Schichtsilikats mindestens 0,3 Gew.-%, vorzugsweise mindestens 0,5 Gew.-%, noch bevorzugter mindestens 0,8 Gew.-%, bezogen auf das Gesamtgewicht der Zusammensetzung, beträgt.

7. Die Zusammensetzung nach einem der vorherigen Ansprüche, wobei das Acrylatpolymer Acrylamideinheiten umfasst, wobei vorzugsweise ein Teil der Acrylamideinheiten eine durch ein Ammoniumsalz modifizierte Sulfonsäuregruppe aufweist.

8. Die Zusammensetzung nach einem der vorherigen Ansprüche, wobei die Menge des Acrylatpolymers höchstens 1,0 Gew.-%, vorzugsweise höchstens 0,5 Gew.-%, bezogen auf das Gesamtgewicht der Zusammensetzung, beträgt.

9. Die Zusammensetzung nach einem der vorherigen Ansprüche mit einer dynamischen Viskosität nach ISO 3219 bei einer Temperatur von 23°C von mehr als 50 mPas, vorzugsweise mehr als 100 mPas.

10. Die Zusammensetzung nach einem der vorherigen Ansprüche mit einer Fließgrenze gemäß ISO 3219 bei einer Temperatur von 23°C zwischen 25 und 250 Pa, vorzugsweise zwischen 50 und 150 Pa.

11. Die Zusammensetzung nach einem der vorherigen Ansprüche mit einer Thixotropie nach ISO 3219 bei einer Temperatur von 23°C zwischen 250 und 750 Pa/s.

12. Die Zusammensetzung nach einem der vorherigen Ansprüche, ferner aufweisend ein Poly(dimethyl)siloxanpolymer.

13. Die Zusammensetzung nach einem der vorherigen Ansprüche, ferner aufweisend eine emulgierende Verbindung, wobei die emulgierende Verbindung zumindest eine Komponente ausgewählt aus der Gruppe bestehend aus einem Fettsäurederivat eines Triglycerids, einem Glucosid, einem Lactylat, einem Monoglycerid, einem Fettalkohol, einem Fettsäurederivat eines Polyglycerins und Mischungen davon darstellt.

14. Ein Artikel zur Körperpflege, aufweisend einen textilen Träger sowie eine Zusammensetzung nach einem der vorherigen Ansprüche.

15. Der Artikel nach Anspruch 14 zur Verwendung bei zumindest einer von Befeuchtung oder Hydratation der Haut, Regeneration der Haut, Wiederherstellung der Hautbarriere, Linderung oder Vorbeugung von Hautunreinheiten, Behandlung altersbedingter Hautbeschaffenheit, Linderung oder Vorbeugung von Hautinfektionen, Wundheilung der Haut, Linderung oder Vorbeugung von Dermatitis oder zur Verbesserung der Anti-Akne-Eigenschaften der Haut.

16. Verfahren zur Herstellung eines Artikels zur Körperpflege, aufweisend die folgenden Schritte:
a. Vorsehen
i) eines textilen Trägers;
ii) einer Zusammensetzung nach einem der Ansprüche 1 bis 13; und
b. Imprägnieren des textilen Trägers mit der Zusammensetzung bei einer Temperatur im Bereich von 5°C bis 50°C.

17. Verfahren nach Anspruch 16, wobei Schritt b. im Wesentlichen drucklos durchgeführt wird, vorzugsweise durch Aufsprühen der Zusammensetzung auf den textilen Träger.

## Revendications

1. Composition comprenant au moins un silicate stratifié, un polymère acrylate, un composé conservateur, un composé humectant et de l'eau, dans laquelle la composition comprend en outre de l'acide hyaluronique ou un sel dérivé de celui-ci, dans laquelle le rapport pondéral entre le silicate stratifié et le polymère acrylate est dans la plage de 2,6:1,0 à 4,3:1,0, et la composition présente une thixotropie selon ISO 3219 à une température de 23°C de 100 à 1000 Pa/s.

2. Composition selon la revendication 1, dans laquelle la quantité de l'acide hyaluronique ou du sel dérivé de celui-ci est jusqu'à 0,5 % en poids sur la base du poids total de la composition.

3. Composition selon la revendication 1 ou 2, dans laquelle le poids moléculaire de l'acide hyaluronique ou du sel dérivé de celui-ci est au moins de 800 kDa, et plus préférentiellement d'au moins 1500 kDa.

4. Composition selon l'une quelconque des revendications précédentes, dans laquelle le dérivé de sel de l'acide hyaluronique est choisi parmi au moins un d'un sel de sodium, d'un sel de potassium, et d'un sel de zinc.

5. Composition selon l'une quelconque des revendications précédentes, dans laquelle le silicate stratifié est une smectite, de préférence choisie dans le groupe constitué par la bentonite, l'hectorite, la montmorillonite, la nontronite, la beidellite, la saponite, la stevensite et des mélanges de ceux-ci, plus préférentiellement une bentonite.

6. Composition selon l'une quelconque des revendications précédentes, dans laquelle la quantité du silicate stratifié est au moins 0,3 % en poids, de préférence au moins 0,5 % en poids, plus préférentiellement au moins 0,8 % en poids, sur la base du poids total de la composition.

7. Composition selon l'une quelconque des revendications précédentes, dans laquelle le polymère acrylate comprend des motifs d'acrylamide, de préférence une part des motifs d'acrylamide comprenant un groupe d'acide sulfonique, qui est modifié par un sel d'ammonium.

8. Composition selon l'une quelconque des revendications précédentes, dans laquelle la quantité du polymère acrylate est au maximum de 1,0 % en poids, de préférence au maximum de 0,5 % en poids, sur la base du poids total de la composition.

9. Composition selon l'une quelconque des revendications précédentes, présentant une viscosité dynamique selon ISO 3219 à une température de 23 °C dépassant 50 mPas, de préférence dépassant 100 mPas.

10. Composition selon l'une quelconque des revendications précédentes, présentant une limite d'élasticité apparente selon ISO 3219 à une température de 23°C comprise entre 25 et 250 Pa, de préférence entre 50 et 150 Pa.

11. Composition selon l'une quelconque des revendications précédentes, présentant une thixotropie selon ISO 3219 à une température de 23°C comprise entre 250 et 750 Pa/s.

12. Composition selon l'une quelconque des revendications précédentes, comprenant en outre un polymère poly(diméthyl)siloxane.

13. Composition selon l'une quelconque des revendications précédentes, comprenant en outre un composé émulsifiant, dans laquelle le composé émulsifiant comprend au moins un composant du groupe constitué par le dérivé d'acide gras de triglycéride, un glucoside, un lactylate, un monoglycéride, un alcool gras, un dérivé d'acide gras d'un polyglycérol, et des mélanges de ceux-ci.

14. Article pour soins personnels, comprenant un support textile et une composition selon l'une quelconque des revendications précédentes.

15. Article selon la revendication 14 pour son utilisation dans au moins une d'une humidification ou hydratation cutanée, régénération cutanée, restauration de la barrière cutanée, soulagement ou prévention d'imperfections cutanées, traitement d'états cutanés liés à l'âge, soulagement ou prévention d'infections cutanées, cicatrisation de lésion cutanée, soulagement ou prévention de la dermatite, ou pour améliorer les propriétés anti-acné de la peau.

16. Processus pour préparer un article pour soins personnels, comprenant les étapes de :
a. fourniture
i. d'un support textile ;
ii. d'une composition selon l'une quelconque des revendications 1 - 13 ; et
b. imprégnation du support textile avec la composition à une température dans la plage de 5 C à 50 C.

17. Processus selon la revendication 16, dans lequel l'étape b. est réalisée sensiblement sans pression, de préférence par pulvérisation de la composition sur le support textile.
